# EUROPÄISCHE PATENTSCHRIFT

(11) **EP 1 226 785 B1**
(45) Veröffentlichungstag und Bekanntmachung des Hinweises auf die Patenterteilung: **09.11.2016**
(21) Anmeldenummer: 01101498.2
(22) Anmeldetag: 24.01.2001
(51) Int. Cl.: A61F 2/28, A61F 2/30, A61F 2/34, B24D 7/18, B28D 1/04, A61F 2/46, A61B 17/16

(54) **Formschleifinstrument**
Shaped grinding tool
Outil de rectification de forme

(43) Veröffentlichungstag der Anmeldung: 31.07.2002
(73) Patentinhaber: Karl Storz GmbH & Co. KG, Postfach 230 78503 Tuttlingen (DE)
(72) Erfinder: Draenert, Klaus, 81545 München (DE)
(74) Vertreter: Vossius & Partner Patentanwälte Rechtsanwälte mbB

(56) Entgegenhaltungen:
- EP-A- 0 201 651
- EP-A- 0 574 736
- DE-A- 3 202 193
- DE-U- 9 413 297
- US-A- 5 314 482

## Beschreibung

Die vorliegende Erfindung betrifft ein Schleifinstrumentarium für das mineralisierte Gewebe, wie zum Beispiel Knochen. Um in ein knöchernes Bett künstliche Implantatkomponenten verankern zu können, wird der Knochen im allgemeinen mit sog. Raffelfräsen bearbeitet und das Implantatlager mit diesen vorbereitet; ein solches Vorgehen ist beschrieben in Müller, M. E. (1973): "Technique of Total Hip Replacement" in: Tronzo, R. G. (ed.): "Sugery of the Hip Joint", Philadelphia: Lea & Febiger und in Müller M. E. (1975): "Total Hip Replacement: Planning, Technique and Complications" in: Cruess & Mitchell (eds.): "Surgical Management of Degenerative Arthritis of the Lower Limb", Philadelphia: Lea & Febiger. Diese groben und scharfen Raffelfräsen haben drei entscheidende Nachteile, sie erhitzen den Knochen, zerstören ihn mechanisch, in dem ganze Knochenkompartimente herausgerissen werden und laufen weg in die weicheren Knochenkompartimente, was heißt, daß das Implantatbett am falschen Ort gesetzt wird. Das Trennen von Knochenabschnitten erfolgt heute ganz allgemein durch oszillierende grob- oder feingezahnte Sägeblätter, was den Knochen mechanisch und thermisch schädigt Es fehlt an einer Technik, die Knochen nahezu atraumatisch bearbeitet und beispielsweise exakt das Bett für das Implantat schleift, wohin es geplant worden war, um die Euzentrizität, das ist die exakte Deckung der beiden Rotationszentren der miteinander artikulierenden Gelenkkörper, reproduzierbar wiederherzustellen, den Knochen vital bis in die Randzone erhält und mechanische Zerstörung verhindert.

In den orthopädischen Operationssälen rund um die Welt werden die Probleme der Vorbereitung des knöchernen Bettes unterschiedlich gelöst. In allen wird jedoch ein grob spanabhebendes Verfahren vom Typ der Raffelfräse eingesetzt mit den oben geschilderten Komplikationen. Im Oberschenkelknochen wird in der Regel eine grobe, gerade Handraspel mit dem Hammer eingeschlagen, welche annähernd die Form der späteren Prothese hat. (Charnley, John (1979): "Low Friction Arthroplasty", Berlin, Heidelberg, New York: Springer). Die Zerstörung des knöchernen Bettes ist dabei um so größer, je anatomischer die Raspel gestaltet wurde, oder je motorisierter das System zur Verfügung steht. In der Regel wird nun in der Beckenpfanne (Acetabulum) mit einer Motorraspel gearbeitet, was zu Hitzenekrosen auf der einen Seite und zur Tieferverlagerung des Rotationszentrums auf der anderen Seite, aber auch zu grobmechanischen Verletzungen im Knochenlager führt; der Fehlsitz des Implantates kommt am Beispiel des Beckens dadurch zustande, daß im kompakten und stark verdichteten Pfannendach die Raffelfräse nicht in den Knochen eindringt und das Gerät sich in Richtung auf das os pubis und os ischii abdrängen läßt. Auch sind hier Bohrungen, wie Charnley sie durchgeführt hat (Charnley 1979: "Low Friction Arthroplasty"), sog. Zentrierbohrungen, nicht geeignet, diese drei Komplikationen zu verhindern. Motorisierte Fräsen im Femur wurden schnell wieder verlassen, da soviel Knochensubstanz zerstört wurde, daß eine Verankerung mit Knochenzement nicht mehr möglich war. Extrem frühe Lockerungen waren die Folge. Die heute oft angewandten anatomischen Raffeleisen haben denselben zerstörerischen Effekt und verhindern eine Zementverankerung in diesem knöchernen Bett nahezu vollständig.

Die heute oft angewandten, anatomischen Raffelfräsen müssen aus diesem Grunde ersetzt werden durch ein System, welches über den geraden Zugang zur Markhöhle ein Maximum an intakter spongiöser Knochenstruktur erhalten läßt. Darüber hinaus kommt es bei den normalen Raffelfräsen auch im Oberschenkelknochen zu einer Displazierung, und zwar wird das Prothesenbett von der starken randständigen Spongiosa des antero-medio-dorsalen U-Shape nach lateral abgedrängt; die mediale, kompakte Knochensubstanz drängt die Fräse nach lateral ab.

DE 32 02 193 A1 offenbart ein chirurgisches Knochenschleifinstrument, bei dem der Schleifkopf über eine Kupplung lösbar mit einem Antriebsteil verbunden ist, wobei der Schleifkopf als Hohlzylinder mit abgerundeter Stirnfläche ausgebildet und an der Außen- und/oder Innenwand mit einem Schleifbelag versehen ist, dessen Partikelgröße zwischen 30 und 300µm liegt.

EP 0 574 736 A1 offenbart ein Schleif-/Bohrwerkzeug für Knochenmaterial bestehend aus einem hohlzyliridrischen Werkzeugschaft, der an seinem vorderen Ende einen ringförmigen Schleifkopf trägt. Der Schleifkopf ist an seiner Oberfläche mit Schleifkornmaterial besetzt. Die Stirnfläche des Schleifkopfes ist an ihrem Innenrand und/oder ihrem äußeren Rand scharfkantig ausgeführt. Der Schleifkopf kann Kühlmittelschlitze oder Kühlmittelbohrungen aufweisen.

In der EP 0099371 wurde ein Verfahren erwähnt, welches den Knochen im Naßschleifverfahren bearbeitet und es erlaubt, intakte Knochenzylinder herauszuschleifen. Mit einem solchen, mit Diamanten besetzten Instrumentarium ist es möglich, den Knochen artefaktfrei zu bearbeiten, ohne die Knochensubstanz zu gefährden. Mit den in diesem Patent beschriebenen Instrumenten war es allerdings nicht möglich, ein Implantatbett vorzubereiten, sondern lediglich, intakte Knochenzylinder zu entnehmen. Es ist danach die Aufgabe der Erfindung, ein Instrument zu entwickeln, mit dem Implantat und Implantatbett atraümatisch, d. h. ohne Zerstörung oder Nekrosen, - eine Nekrose bedeutet abgetötetes Gewebe, - zu erzeugen und präzise und positionsgetreu einzupassen sind. Dies ist mit der nachfolgenden Erfindung leicht und vollständig gelungen. Die Erfindung ist durch die Merkmale der Patentansprüche gekennzeichnet.

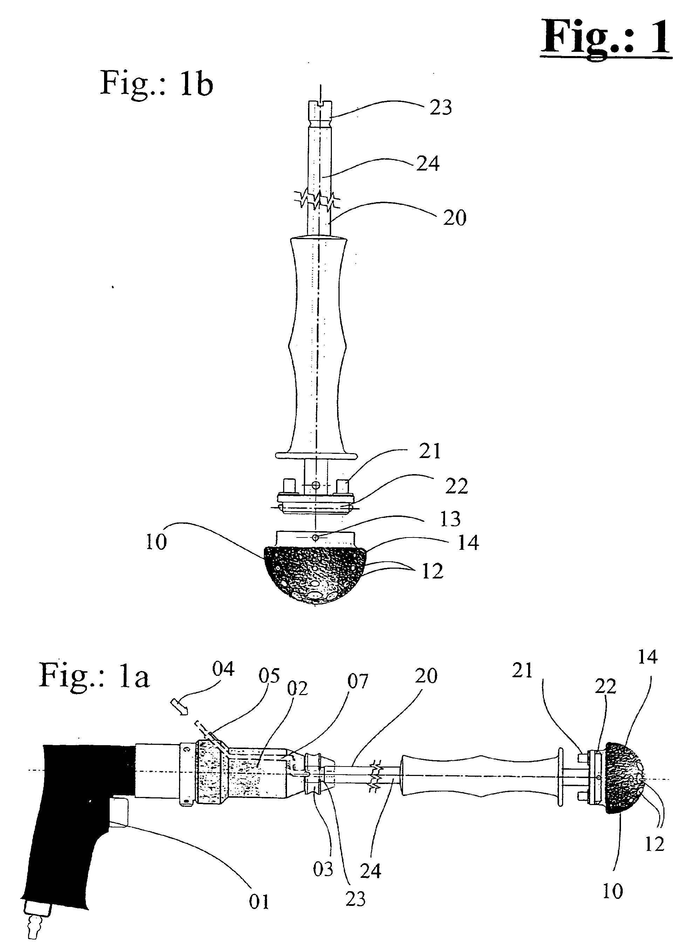

In den Figuren 1a und 1b ist ein einfaches Instrument dargestellt, bestehend aus einer starken Antriebsmaschine 01 und einem Adapter (02 in einem schematischen Längsschnitt. Das Verlängerungsstück 20 mit integrierter Spülleitung 24 ist bei 23 in der Antriebsmaschine eingekoppelt. Aufgesetzt und mit einer Schnellkupplung (21, 22 fixiert ist ein hohler Schleifkörper 10, besetzt mit einem groben Diamant 14 (425 µm) in galvanischer Bindung mit Öffnungen 12 zum Austritt der Spüllösung.

Die Antriebsmaschine 01 entwickelt ein Drehmoment, welches den Flächenreibungswiderstand des Schleifkopfes im knöchernen Lager einer Hüftgelenkspfanne überwindet und den Schleifkopf mit hoher Drehzahl auf dem Knochen schleifen läßt. Angeschlossen über den Adapter 02 ist eine Perfusionslösung, in der Regel Ringerlaktat 04, welche mit einem Luerlock 05 an den Adapter angeschlossen ist und über die integrierte Spülleitung 07 in das Verlängerungsstück 20/24 gelangt und aus den Öffnungen 12 zwischen Knochen und Diamantschicht austritt. Der diamantbeschichtete Schleifkopf bleibt exakt dort plaziert, wo er aufgesetzt wird und schleift den mineralisierten Knochen, ohne Knochenbälkchen oder Markgewebe herauszureißen. Während des Schleifens wird der Knochenabrieb ohne Hitzeentwicklung weggespült und aufgesaugt; der Diamantschleifbelag wird auf diese Weise nicht mit Knochenmehl zugeschmiert. Das geschliffene Knochenbett ist danach so präzise vorbereitet, daß eine press-fit Pfanne aus Titan mit einer Oberflächenrauhigkeit rₜ = 80 µm - 100 µm mit einer Differenz zwischen 600 µm und 1000 µm, vorzugsweise um 800 µm in das elastische Knochenbett verpreßt werden kann. Der Schleifbelag 14 auf dem Formschleifkörper 10 besitzt eine Dicke von 300 µm bis 900 µm, vorzugsweise um 500 µm. Die Schleifpartikel haben eine Größe zwischen 60 µm und 800 µm, sind hart und scharfkantig, und bis auf eine Tiefe von 30-80 %, vorzugsweise 50-60 % in eine vorzugsweise galvanisch aufgetragene Bindung eingebettet. Eine optimale Korngröße für konvex/konkave, sphärische Schleifkörper ist beispielsweise das konfektionell erhältliche Diamantkorn von 426 µm. Der Belag wird in üblicher Weise aufgetragen, z. B. durch galvanische Beschichtung.

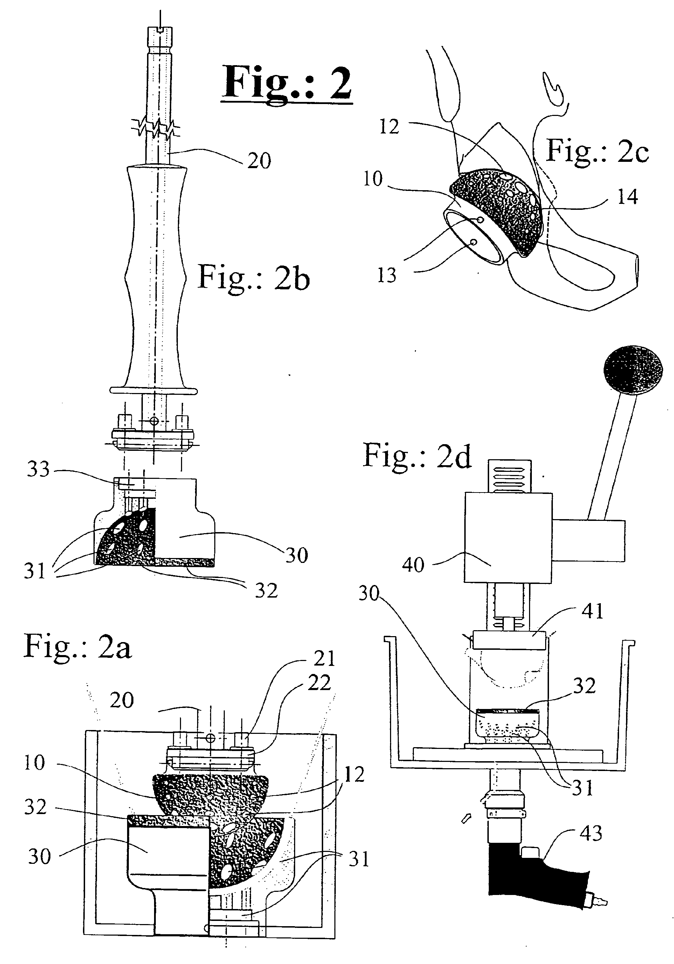

In den Figuren 2a-2d von Figur 2 ist eine Vorrichtung dargestellt, die ein altes Problem der Hüftchirurgie löst, in dem nämlich optimal nur dann eine Knochenzementverankerung erreicht werden kann, wenn über den Spongiosaknochen auf die kompakte Knochenrinde zu die Aussteifung mit Knochenzement erfolgt; dies ist im Oberschenkelknochen natürlicherweise so der Fall, nicht jedoch in der Pfanne des Beckens: Im Becken wird die Knochenzementauffüllung über Bohrungen durch die Knochenrinde in enge und darauffolgend weiter werdende Spongiosawaben durchgeführt, was zwangsläufig zu unvollständigen Aussteifungen der Waben führen muß. Mit der in den Figuren 2a - 2d dargestellten Vorrichtung kann dieses Problem sehr elegant zum Nutzen des Patienten gelöst werden.

Die Figuren 2a-2d von Figur 2 zeigen ein Beispiel zur Herstellung einer Knochenschale als Bett für die Implantatverankerung.

Der bei einer Hüftgelenkersatzoperation abgesetzte Kopf mit dem dazugehörigen Halsanteil (Kopf-Hals-Knochen) wird in einer Schleifvorrichtung über eine Haltevorrichtung 40, 41 fixiert und mit Steinmann-Nägeln stabil mit der Haltevorrichtung 40,41 verbunden, die wiederum den fixierten Kopf über einen Kniehebel in den rotierenden Teller, der mit einer rotierenden Hohlschleife bestückt ist, gepreßt werden kann. Das konkave Formschleifinstrument 30 ist sphärisch gestaltet und am Boden mit Durchtrittsöffnungen 31 für eine unter Druck austretende Spülflüssigkeit versehen. Die Spülflüssigkeit spült den Knochenabrieb zwischen Diamant und Knochen weg und kühlt gleichzeitig den Knochen, indem die Reibungswärme abgeführt wird. Auf diese Weise wird der Diamantbelag 32 frei von Debris-Material gehalten und der Kochen nicht thermisch geschädigt. Über die Kniehebelpresse wird der Femurkopf mit dem Hals-Anteil in den Formschleifkörper hereingepreßt und mit hoher Drehzahl z. B. über einen Bohrmaschinenantrieb 43 geschliffen, bis seine Spongiosawabenstrukturen unterhalb der Knorpelgrundplatte über die gesamte Fläche erkennbar und weit offen dargestellt sind. Ist die konvexe Seite des Femurkopfes in dieser Weise geschliffen und sind die Spongiosawaben weit offen dargestellt, wird die Antriebsmaschine umgesteckt und die gegenüberliegende Seite des Knochens mit einem sphärischen, konvexen Formschleifkörper 10 mit hoher Drehzahl geschliffen (Fig. 2a). Die Dicke der so entstehenden Knochenschale ist definiert durch die Differenz der Durchmesser zwischen der konkaven und der konvexen Fräse. Diese wird in einer präoperativen Planung am Röntgenbild festgelegt und richtet sich nach der Rekonstruktion des Rotationszentrums unter Berücksichtigung einer standardisierten HDPE-Pfannenschale, welche in die Knochenschale einzementiert werden kann.

Zur Vorbereitung im Beckenknochen wird mit einer konvexen Hohlschleife das knöcherne Bett geschliffen (Fig. 2c).

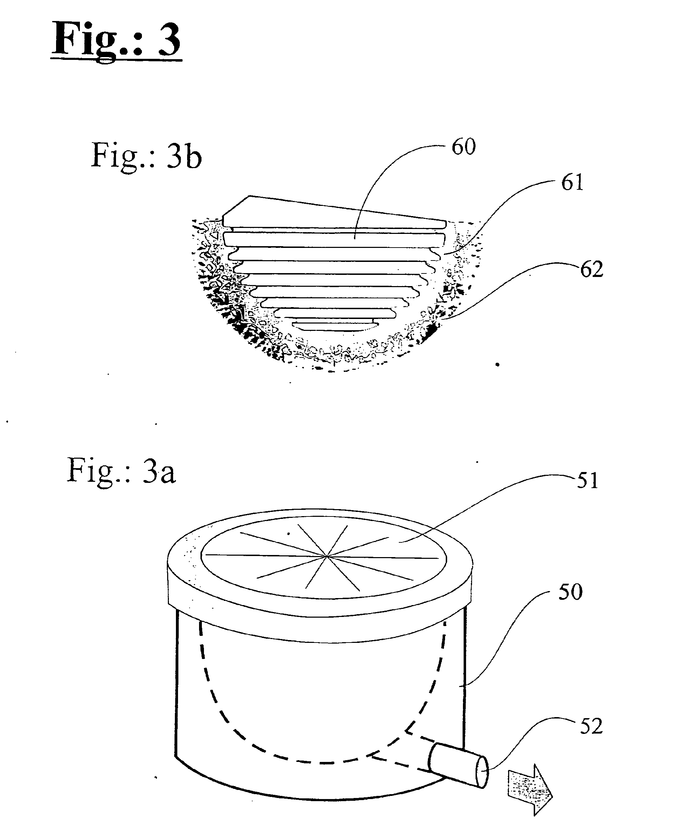

Die sauber von beiden Seiten geschliffene Knochenschale wird anschließend mit 5 %-iger H₂0₂-Lösung gewaschen und in ein besonderes Gefäß 50 (Fig. 3a und b), fest in eine Silikondichtung 51 eingepreßt, danach wird Knochenzement angerührt und der standardvisköse, unter Vakuum angerührte und daher blasenfreie Knochenzement wird unter Vakuum, welches an die Vakuumapplikationsvorrichtung 52 angelegt wird, in die Knochenschale eingesaugt und danach eine HDPE-Pfanne in die Knochenschale eingedrückt, der überstehende Knochenzement wird sorgfältigst entfernt und das Knochen-Pfannen-Implantat auf diese Weise für das Einsetzen präpariert (Fig. 3b) und (Fig. 2c). In der Fig. 3 ist das Ergebnis dargestellt: Die z. B. HDPE-Kunststoffpfanne 60 ist danach über den Knochenzement 61 tief in der Knochenstruktur verankert (Knochen, geschnitten: 62).

Bevor nun dieses in dieser Weise präparierte Implantat eingesetzt wird, muß mit einer ebenfalls konvexen, sphärischen Formschleife, welche sich im Außendurchmesser um 600 µm bis 1000 µm, vorzugsweise um 800 µm vom Innendurchmesser der konkaven Schleifvorrichtung unterscheidet, in der Weise, daß sie kleiner ist als die konkave Formschleife, der Knochen vorbereitet werden (Fig. 2c). Die konvexe, sphärische Formschleife 10 wird in oben beschriebener Neigung (38°) und Anteversion (8°-12°) das knöcherne Lager vorbereiten, in dem dieses geschliffen wird, bis der Rand der Schleife in der Tiefe des Acetabulums hinter dem Rand des Acetabulums verschwindet. Nach der gründlichen Reinigung der Knochenwaben mit der Jet-Lavage wird eine Spülung mit Heparin angeschlossen (10.000 I. E. in 200 ml Ringerlösung). Danach wird das Knochen-Pfannen-Implantat mit dem Setzinstrument im korrekten Winkel von 38 ⁰ seitlicher Neigung und 8 ⁰ bis 12 ⁰ Anteversion in das Pfannenlager eingeschlagen. Der knöcherne Press-Sitz reicht in aller Regel für die stabile, primäre Fixation aus; das Implantat, welches aus der mit Knochenzement ausgesteiften Schale besteht, in die das Implantat bereits einzementiert ist, kann aber auch mit 1-4 Knochendübeln oder Schrauben stabil verankert werden.

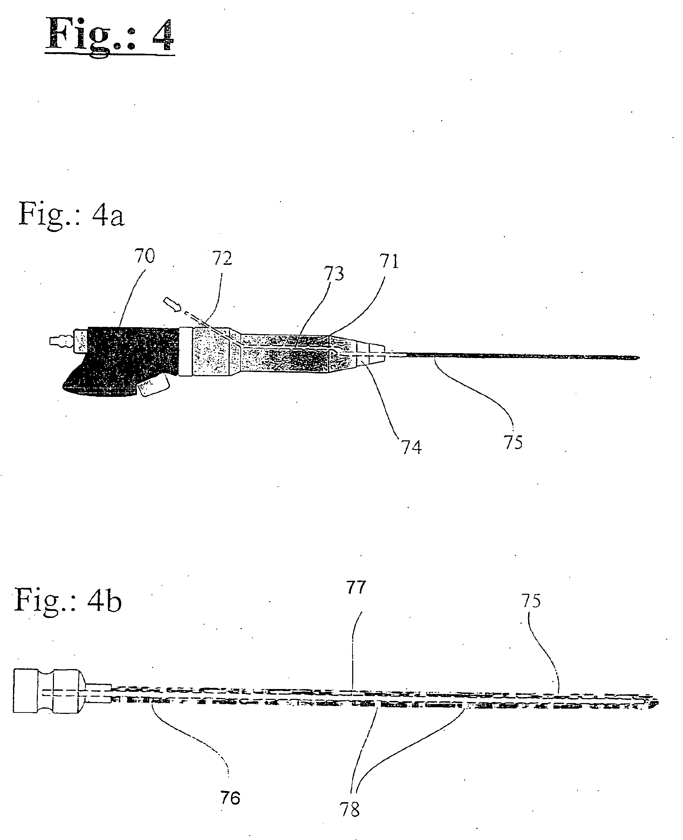

Ein weiteres Beispiel eines Knochentrennschleifinstrumentes ist in den Fig. 4a und b dargestellt mit einer hochtourig drehenden Antriebsmaschine 70, beispielsweise einer pressluftgetriebenen Luftturbine mit ausreichendem Drehmoment, welche über eine integrierte Spülleitung in einem Adapterstück 71 die Möglichkeit eröffnet, über beispielsweise einen Luer-Lock Anschluß 72 durch den Spülkanal 73 einem mit Schnellkupplung 74 eingesetzten Instrument 75, welches außen und über die Spitze mit Diamant 76 beschichtet ist, Spüllösung zuzuführen, wobei dessen Beschichtung über den integrierten Spülkanal 77 von innen über Öffnungen 78 im Belag freigehalten wird und wobei die erzeugte Reibungswärme über die Spüllösung abgeführt wird.

## Patentansprüche

1. Instrument zur Vorbereitung eines knöchernen Implantatbettes und/oder Bearbeitung von Knochen mit einem rotierenden und/oder oszillierenden. Formkörper, der fest oder über eine Kupplung (21, 22) lösbar mit einem Antriebsteil (01) verbindbar ist und an seiner, zum Knochen zugewandten Oberfläche dkekt als Schleifkörper (10) ausgebildet ist oder über seine Außenfläche breit mit einem Schleifbelag (14) besetzt ist, mit dem der Knochen entsprechend dem Formkörper geschliffene und/oder getrennt werden kann, wobei der direkt als Schleifkörper ausgebildete Formkörper oder der mit einem Schleifbelag besetzte Formkörper mehrere Durchtrittsöffnungen (12, 31, 78) für eine Spülflüssigkeit aufweisen, **dadurch gekennzeichnet, daß** die dem Knochen zugewandte Oberfläche des Formkörpers sphärisch konvex oder sphärisch konkav oder daß der Formkörper als kompakter zylinder oder hohler Zylinder welcher mit einer dem Knochen zugewandten Spitze gechlossen ist, ausgebildet ist, wobei der Hohlzylinder außen und über seine dem Knochen zugewandte Spitze mit dem Schleifbelag beschichtet ist.

2. Instrument nach Anspruch 1, wobei der Formkörper hohl ist und/oder von Spülkanälen durchzogen ist zum Führen der Spülflüssigkeit durch die Öffnungen zwischen Knochen und Schleifbelag.

3. Instrument nach den Ansprüchen 1 und 2, wobei der Schleifbelag natürlichen oder künstlichen Diamant, ein Hartmetall, Al₂O₃ oder ähnlich harte Partikel oder ein Partikelgemisch aus verschiedenen harten und scharfen Partikeln verschiedener Materialien, welche zum Schleifen, von Knochen geeignet sind, aufweist.

4. Instrument nach Anspruch 3, wobei der Schleifbelag scharfkantige Partikel zwischen 60 µm und 800 µm, vorzugsweise um 100 µm bis 450 µm aufweist.

5. Instrument nach den Ansprüchen 1 bis 4, wobei der Formkörper als Halbkugel oder Kugelsegment, als Halbhohlkugel oder als Hohlkugelsegment mit seiner konkaven Oberfläche oder mit seiner konvexen Oberfläche zum Knochen ausgerichtet ist und jeweils mit seiner Außen- oder Innenfläche dem Knochen zugewandt, diesen schleifen kann.

6. Instrument nach den Ansprüchen 1 bis 5, wobei mindestens eine Durchtrittsöffnung einen Durchmesser zwischen 0,2 bis 5 mm in Form von Bohrungen und/oder Schlitzen aufweist, die in die Schleiffläche münden.

7. Instrument nach den Ansprüchen 1 bis 6, mit einem Kupplungsteil, das als Verlängerungsstück ausgebildet ist und eine röhrenförmig Achse darstellt, welche selbst wiederum über eine Kupplung mit dem Antriebsteil lösbar oder fest verbindbar ist und über die Spülflüssigkeit von innen in den als Hohlschleifkörper ausgebildeten Formkörper nach außen auf die beschichtete Oberfläche des Hohlschleifkörpers unter Druck geführt werden kann.

8. Instrument nach Anspruch 7, wobei eine tellerförmige Schnellkupplung eine lösbare Koppelung des Verlängerungsstücks an den Formkörper erlaubt.

9. Instrument nach den Ansprüchen 1 bis 8, wobei der Schleifbelag mit den. Schleifpartikeln mit Hilfe einer galvanischen Beschichtung eine Einbett-Tiefe von 30 bis 80% der Partikelgröße aufweist, vorzugsweise eine Einbett-Tiefe von 50 bis 60 %.

10. Instrument nach den Ansprüchen 1 bis 9, wobei ein mit Schleifbelag versehener halbkugelförmiger Formkörper oder ein Kugelsegment, dessen konkave Fläche mit einem Schleifbelag beschichtet ist, in Kombination mit einem sphärischen Formkörper, dessen konvexe Fläche mit diesem Belag beschichtet ist, in der Weise zusammenwirken, dass ein Knochen, zwischen beiden Formkörpern gehalten, von der einen Seite konvex und von der anderen Seite konkav bearbeitet werden kann und auf diese Weise eine Knochenschale geschliffen werden kann.

11. Kombination von Instrumenten der Ansprüche 1 bis 10 bestehend aus zwei konvexen, sphärischen Formkörpern in Kombination mit einem konkaven, sphärischen Formkörper, wobei
- der kleinere der konvexen Formkörper so ausgebildet ist, dass er in ein gehaltenes Knochenstück, z. B. einen Femurkopf, eine konkave, sphärische Vertiefung schleifen kann,
- der konkave Formkörper so ausgebildet ist, dass er die Außenseite einer entstehenden Knochenschale schleifen kann,
- wobei die Differenz zwischen dem Durchmesser des konkaven. Formkörpers und dem Durchmesser des kleineren konvexen Formkörpers die Dicke der Knochenschale bestimmt
- der größere konvexe Formkörper so ausgebildet ist, dass er ein Implantatbett im Knochen schleifen kann und sein Durchmesser etwas kleiner ist als der Durchmesser des konkaven Formkörpers,
- so dass die Knochenschale press-fit in das geschliffene Knochenbett eingesetzt werden kann.

## Claims

1. A tool for preparing a bony implant bed and/or for machining bones by means of a rotating and/or oscillating molded member being permanently or detachably connectable to a driving part (01) by means of a coupling (21, 22) and being either directly designed as a grinding tool (10) on the surface facing the bone or covered with a broad grinding layer (14) on its outer surface in order to grind and/or split the bone according to the molded member, wherein the molded member directly designed as a grinding tool or the molded member covered with a grinding layer comprises a plurality of openings (12, 31, 78) for a rinsing liquid, **characterized in that** the surface of the molded member facing the bone is formed spherically convex or spherically concave or that the molded member is formed as a compact cylinder or hollow cylinder closed with a tip facing the bone, wherein the hollow cylinder is covered with the grinding layer on the outside and via the tip facing the bone.

2. The tool according to claim 1, wherein the molded member is hollow and/or has rinsing channels running through it for guiding the rinsing liquid through the openings between the bone and the grinding layer.

3. The tool according to claims 1 and 2, wherein the grinding layer comprises natural or synthetic diamond, cemented carbide, Al₂O₃ or similarly hard particles or a particle mixture of various hard and sharp particles of various materials suitable for grinding bones.

4. The tool according to claim 3, wherein the grinding layer comprises sharp-edged particles having a size between 60 µm and 800 µm, preferably between 100 µm to 450 µm.

5. The tool according to claims 1 to 4, wherein the molded member, as a hemisphere or spherical segment, a hollow hemisphere or hollow spherical segment, is directed to the bone with its concave surface or its convex surface and able to grind it with its outer or inner surface facing the bone.

6. The tool according to claims 1 to 5, wherein at least one opening comprises a diameter between 0.2 and 5 mm in the form of drilling holes and/or slots opening out into the grinding surface.

7. The tool according to claims 1 to 6 comprising a coupling element designed as an extension piece and representing a tubular axle which in turn is permanently or detachably connectable to the driving part by means of a coupling and via which rinsing liquid can be guided with pressure from the inside into the molded member designed as a hollow grinding tool to the outside onto the coated surface of the hollow grinding tool.

8. The tool according to claim 7, wherein a disc-shaped quick-action coupling allows for the extension piece to be detachably coupled to the molded member.

9. The tool according to claims 1 to 8, wherein the grinding layer with the grinding particles comprises an embedment depth of 30 to 80 %, preferably of 50 to 60 %, of the particle size with the help of an electroplated coating.

10. The tool according to claims 1 to 9, wherein a hemispherical molded member coated with a grinding layer or a spherical segment whose concave surface is coated with a grinding layer interacts with a spherical molded member whose convex surface is coated with said layer in such a way that a bone which is clamped between said two molded members can be machined convexly from the one side and concavely from the other side and that a bone shell can be ground in this manner.

11. A combination of the tools according to claims 1 to 10 consisting of two convex, spherical molded members in combination with a concave, spherical molded member, wherein
- the smaller one of the convex molded members is designed in such a way that it can grind a concave, spherical recess into a clamped piece of bone, e.g., a femoral head,
- the concave molded member is designed in such a way that it can grind the outer surface of a bone shell to be produced,
- wherein the balance of the diameter of the concave molded member and the diameter of the smaller convex molded member determines the thickness of the bone shell,
- the larger convex molded member is designed in such a way that it can grind an implant bed in the bone and that its diameter is slightly smaller than the diameter of the concave molded member,
- so that the bone shell can be inserted in a press-fit way into the ground bone shell.

## Revendications

1. Instrument de préparation d'un site implantaire osseux et/ou de travail d'os, comprenant un corps moulé rotatif et/ou oscillant, qui peut être solidarisé à demeure ou de manière libérable par l'intermédiaire d'un accouplement (21, 22) à une unité d'entraînement (01) et qui est réalisé directement sous la forme d'un corps abrasif (10) au niveau de sa surface tournée vers l'os, ou dont toute la surface extérieure est dotée sur sa largeur d'une garniture abrasive (14) au moyen de laquelle l'os peut être rectifié et/ou fendu en fonction du corps moulé, le corps moulé réalisé directement sous la forme d'un corps abrasif ou le corps moulé doté d'une garniture abrasive comprenant plusieurs orifices de passage (12, 31, 78) pour un liquide de rinçage, **caractérisé en ce que** la surface du corps moulé tournée vers l'os est sphériquement convexe ou sphériquement concave ou **en ce que** le corps moulé est réalisé sous la forme d'un cylindre compact ou d'un cylindre creux, lequel est fermé au moyen d'une pointe tournée vers l'os, l'extérieur du cylindre creux et toute l'étendue de sa pointe tournée vers l'os étant revêtus de la garniture abrasive.

2. Instrument selon la revendication 1, le corps moulé étant creux et/ou traversé par des canaux de rinçage servant à guider le liquide de rinçage à travers les orifices situés entre l'os et la garniture abrasive.

3. Instrument selon les revendications 1 et 2, la garniture abrasive comprenant du diamant naturel ou artificiel, un métal dur, Al₂O₃ ou des particules similairement dures ou un mélange de particules composé de différentes particules dures et tranchantes de différents matériaux, lesquels sont adaptés à la rectification d'os.

4. Instrument selon la revendication 3, la garniture abrasive comprenant des particules à arêtes vives comprises entre 60 µm et 800 µm, de préférence de 100 µm à 450 µm.

5. Instrument selon les revendications 1 à 4, le corps moulé étant réalisé sous la forme d'une demi-sphère ou d'un segment sphérique, d'une demi-sphère creuse ou d'un segment sphérique creux, sa surface concave ou sa surface convexe étant orientée vers l'os, ledit corps pouvant rectifier l'os, sa surface extérieure ou intérieure étant dans chaque cas tournée vers l'os.

6. Instrument selon les revendications 1 à 5, au moins un orifice de passage présentant un diamètre compris entre 0,2 et 5 mm sous forme de trous et/ou de fentes qui débouchent dans la surface abrasive.

7. Instrument selon les revendications 1 à 6, comprenant une partie d'accouplement qui est réalisée sous la forme d'une pièce de rallonge et qui présente un axe tubulaire, lequel peut être solidarisé pour sa part à demeure ou de manière amovible au moyen d'un accouplement à la partie d'entraînement et à travers lequel le liquide de rinçage peut être guidé sous pression depuis l'intérieur dans le corps moulé réalisé sous la forme d'un corps abrasif creux vers l'extérieur sur la surface revêtue du corps abrasif creux.

8. Instrument selon la revendication 7, un accouplement rapide en forme de plateau permettant un accouplement libérable de la pièce de rallonge sur le corps moulé.

9. Instrument selon les revendications 1 à 8, la garniture abrasive comportant les particules abrasives présentant à l'aide d'un revêtement galvanique une profondeur d'implantation de 30 à 80 % de la taille des particules, de préférence une profondeur d'implantation de 50 à 60 %.

10. Instrument selon les revendications 1 à 9, un corps moulé semi-sphérique pourvu d'une garniture abrasive ou un segment sphérique, dont la surface concave est revêtue d'une garniture abrasive, coopérant avec un corps moulé sphérique, dont la surface convexe est revêtue de cette garniture, de telle manière qu'un os, maintenu entre les deux corps moulés, peut être travaillé de manière convexe par l'un des côtés et de manière concave par l'autre côté, et une coque osseuse peut ainsi être rectifiée.

11. Combinaison d'instruments selon les revendications 1 à 10, constituée de deux corps moulés sphériques convexes combinés à un corps moulé sphérique concave,
- le plus petit des corps moulés convexes étant réalisé de telle manière qu'il peut ménager par rectification un creux sphérique concave dans un morceau d'os maintenu, par exemple une tête de fémur,
- le corps moulé concave est réalisé de telle manière qu'il peut rectifier la face extérieure d'une coque osseuse ainsi produite,
- la différence entre le diamètre du corps moulé concave et le diamètre du plus petit corps moulé convexe détermine l'épaisseur de la coque osseuse,
- le plus grand corps moulé convexe est réalisé de telle sorte qu'il peut rectifier un site d'implantation dans l'os et son diamètre est un peu inférieur au diamètre du corps moulé concave,
- de sorte que la coque osseuse peut être insérée par ajustement avec serrage dans le site osseux rectifié.
